# EUROPEAN PATENT SPECIFICATION

(11) **EP 4 240 437 B1**
(45) Date of publication and mention of the grant of the patent: **23.09.2026**
(21) Application number: 21889984.7
(22) Date of filing: 03.11.2021
(51) Int. Cl.: A61L 27/36, A61L 27/50, A61F 2/00

(54) **MESHED PLACENTAL MEMBRANE TISSUE GRAFTS**
VERMASCHTE PLAZENTAMEMBRANGEWEBETRANSPLANTATE
GREFFES DE TISSU DE MEMBRANE PLACENTAIRE MAILLÉES

(30) Priority: 05.11.2020 US 202063109980 P
(43) Date of publication of application: 13.09.2023
(73) Proprietor: MIMEDX Group Inc., Marietta, GA 30062 (US)
(72) Inventor: MCQUEEN, Adam, Marietta, Georgia 30062 (US); LEVAUGHN, Rick, Marietta, North Carolina 30062 (US); KOOB, Thomas J., Marietta, Georgia 30062 (US); MASSEE, Michelle, Marietta, Georgia 30062 (US)
(74) Representative: Murgitroyd & Company
(86) International application number: PCT/US2021/057893
(87) International publication number: WO 2022/098755

(56) References cited:
- US-A1- 2005 021 141
- US-A1- 2013 084 314
- US-A1- 2013 084 314
- US-B1- 6 326 019
- US-B2- 8 323 701

## Description

### FIELD OF THE INVENTION

The invention relates to expandable, dehydrated, meshed placental membrane tissue allografts that can be rehydrated and expanded to cover a variety of wounds having an irregular shape.

### BACKGROUND

Human placental membrane (e.g. amniotic membrane or tissue) has been used for various types of reconstructive surgical procedures since the early 1900s. The membrane serves as a substrate material, more commonly referred to as a biological dressing or patch graft. Such membrane has also been used widely for ophthalmic procedures in the United States and in countries in the southern hemisphere. Typically, such membrane is either frozen or dried for preservation and storage until needed for surgery.

Such placental tissue is typically harvested after an elective Cesarean surgery. The placenta has two primary layers of tissue including amniotic membrane and chorion, separated by an intermediate or spongy layer. The amniotic membrane is a non-vascular tissue that is the innermost layer of the placenta, and consists of a single layer, which is attached to a basement membrane. Histological evaluation indicates that the membrane layers of the amniotic membrane consist of epithelium cells, thin reticular fibers (basement membrane), a thick compact layer, and fibroblast layer. The fibrous layer of amnion (i.e., the basement membrane) contains cell anchoring collagen types IV, V, and VII. The intermediate layer consists of a proteoglycan-rich substance that serves as a lubricant between the amnion and chorion. The chorion is also considered as part of the fetal membrane; however, the amniotic layer and chorion layer are separate and separable entities. The chorion consists of a reticular layer, a basement membrane, and a trophoblast cell layer, in order; *in vivo,* the reticular layer is adjacent to the intermediate layer.

Amniotic membranes provide unique grafting characteristics when used for surgical procedures, including providing a matrix for cellular migration/proliferation, providing a natural biological barrier, are non-immunogenic, promote increased self-healing, are susceptible of being fixed in place using different techniques including fibrin glue or suturing. And, such grafts, when properly prepared, can be stored at room temperature for extended periods of time, without need for refrigeration or freezing, until needed for a surgical procedure.

Known clinical procedures or applications for such placental membrane grafts include Schneiderian membrane repair (i.e. sinus lift), guided tissue regeneration (GTR), general wound care, and primary closure membrane. Known clinical procedures or applications for such placental membrane grafts include biological would dressing.

A detailed look at the history and procedure for harvesting and using "live" amniotic tissue for surgical procedures and a method for harvesting and freezing amniotic tissue grafts for ophthalmic procedures is described in U.S. Pat. No. 6,152,142 issued to Tseng. US 8 323 701 B2 discloses tissue grafts derived from the placenta. The grafts are composed of at least one layer of amnion tissue where the epithelium layer has been substantially removed in order to expose the basement layer to host cells. In one embodiment, there is provided a tissue graft comprising a first membrane comprising an amnion and one or more additional membranes laminated to the first membrane, wherein the first membrane comprises an exposed basement membrane and stromal layer, and the one or more additional membranes are adjacent to the stromal layer. One or more additional membranes are selected from the group consisting of amnion, chorion, or a combination thereof. After the dehydration phase, the tissue graft is perforated using a small gauge needle, or punch to produce a mesh-type configuration.

However, placental membrane tissue is generally limited by its size and dimension, and not amenable to use to treat larger or unusually shaped wounds. The subject matter described herein addresses the shortcomings.

### SUMMARY OF THE INVENTION

The subject matter described herein is directed toward a meshed, dehydrated and sterile, placental membrane tissue graft, and particularly human allograft, that is expandable to cover the shape of a wound site that is extensive and often irregularly shaped. It is important to note that a placental membrane tissue graft can be comprised of single layers of amnion or chorion, multiple layers of amnion or chorion, or multiple layers of a combination of amnion and chorion. The invention is defined in the appended claims.

The invention provides a dehydrated, meshed, expandable, and sterile, placental tissue allograft comprising a plurality of pre-determined cuts in an engineered pattern of cuts, wherein said cuts are slits. The allograft is expandable by at least 100% of its original size when rehydrated in 0.9% saline solution. The cuts permit expansion of the graft without significantly compromising its structural integrity. These cuts or incisions may have been incorporated into the graft after the tissue has been decontaminated and dehydrated. Based on the particular embodiment, the cuts can be applied by a commercially available meshing device, a laser cutting device, or a cutting template. Additionally, the dehydration step may be carried out by well-known techniques in the art, such as air drying, chemical drying, or lyophilization of the placental membrane tissues. Additionally, certain embodiments comprise a mesh pattern which enhances the expansion capabilities of the dehydrated placental membrane tissue graft. In other embodiments, multiple, unique mesh patterns may be applied to a single graft. The placental tissue allograft is contained within a sealed pouch.

After harvesting, the placental membrane tissue is treated in a number of steps to provide the products described herein. For example, amniotic membrane and the chorion layers are separated from other placental tissue, such as the umbilical cord and the placental disc tissue (the other tissues may be retained for other purposes). All components are sourced from a single donor. The placental membrane tissue is subject to a specific process in which it is rinsed in a salt solution, and then rinsed again in an antibiotic solution, and then a final rinse in yet another salt solution designed to remove residual antibiotic solution from the tissue.

The placental membrane tissue may be initially cleaned in a hyperisotonic solution wherein the hyperisotonic solution comprises NaCl concentration in a range of from about 30% to about 10%.

Also disclosed (not claimed) is a method further comprising the step of separating the chorion tissue layer from the amniotic membrane layer. In some examples, the method further includes the step of, after separation of the chorion tissue layer from the amniotic membrane layer, physically cleaning the selected layer to remove blood clots and other contaminates. After separating the selected layers, they may be soaked in an antibiotic solution. Optionally, the method then also includes the step of rinsing the selected layer to remove residual antibiotic solution.

Also disclosed (not claimed) is a method that involves keeping the amnion and chorion layers intact, without separating the layers and removing the intermediate layer. The native amnion and chorion layers are then subjected to physical cleaning to remove blood clots and subsequent rinsing in an antibiotic solution. Additionally, the intact layers are subject to another rinsing step to remove residual antibiotic solution.

The amniotic tissue is gently cleansed and minimally manipulated to preserve inherent growth factors and proteins in the tissue. Notable growth factors in the amniotic tissue include transforming growth factor beta (TGF-β), basic fibroblast growth factor (bFGF), platelet derived growth factors (PDGF AA & BB), and vascular endothelial growth factor (VEGF)14,15, which are known to regulate the healing cascade.

After the rinsing and decontamination steps have been carried out, the selected layers of placental tissue are then subjected to a drying process, which may involve any type of commercially acceptable process known in the art, including, but not limited to air drying, chemical drying or lyophilization of the placental cord tissue.

In one embodiment, the selected tissue layers are dried on a fixture. The surface of the drying fixture has a plurality of grooves that defines the outer contours of each of the plurality of placenta membrane tissue grafts and wherein the step of cutting comprises cutting the selected layer along the grooves.

After the selected layers of placental tissue have been dried and cut to the desired size and shape, a specific cut pattern is then applied which gives the tissue a meshed appearance. Based on the desired embodiment, the cut pattern can be varied, and may be applied through the use of commercially available apparatuses, such as a meshing tool, a laser cutting tool or a cutting template.

The finished product is packaged in a sterile container and is reconstituted with an acceptable excipient by the end user before the graft is applied to the subject's wound site. Alternatively, the graft can be applied directly to the wound site and can be reconstituted with a combination of an excipient and the patient's own bodily fluids that may be present in the wound site.

Also disclosed (not claimed) is a method for forming an expandable placental tissue graft having structural integrity which method comprises a) obtaining a dehydrated placental tissue graft; b) placing a set of cuts or incisions into said graft to permit expansion of the graft; and c) sterilizing said graft.

### BRIEF DESCRIPTION OF THE FIGURES

Further features and benefits of the present invention will be apparent from a detailed description of preferred embodiments thereof taken in conjunction with the following drawings, wherein similar elements are referred to with similar reference numbers, and wherein:
Fig. 1 illustrates a slit mesh pattern prototype very similar in pattern to the slits achieved by a traditional skin graft mesher, but the slit lengths and spacing are set to achieve peak expansion of the meshed graft. The expanded graft will have a 2.5 mm thick border on the long edge of the graft and 1.5 mm thick strands of tissue surrounding large holes where incisions are made. The tissue will expand along the short axis of the graft. Both laser and die cut methods of manufacturing can be utilized to incorporate this pattern.
Fig. 2 illustrates a zig zag mesh pattern. When expanded, this mesh pattern will create small holes with strips of tissue covering each small hole. This mesh pattern will result in tissue strands that are diagonally positioned and very close together allowing the graft to maintain its structural integrity during handling and suturing.
Fig. 3 illustrates a spiderweb mesh pattern created by positioning the slit design at different angles from the center of the graft to create a hexagonal effect.
Fig. 4 illustrates an "evil eye" mesh pattern comprising a dense series of straight and slanted slits.
Fig. 5 illustrates a modified version of the evil eye mesh pattern designed to create thicker strands of tissue compared to the original evil eye mesh pattern. The number of slits is reduced significantly in relation to this mesh pattern.
Fig. 6 illustrates another evil eye mesh pattern designed to create thicker strands of tissue, which allows for a greater degree of expansion. The number of slits is reduced slightly in comparison with the other two evil eye mesh patterns.
Fig. 7 illustrates the dehydrated, meshed, placental tissue graft in its native state, prior to reconstitution or expansion.
Fig. 8 illustrates the reconstituted, meshed placental cord graft after a desired excipient has been applied, and force has been applied, causing the graft to expand.

### DETAILED DESCRIPTION OF THE INVENTION

One challenge that has been encountered in the field is that placental tissue grafts (as well as all tissue grafts in general) are of a uniform shape and size, even though wounds tend to be irregularly shaped. Placental tissue allograft sizes are constrained by the dimensions of the source placental membrane tissue itself; therefore, larger wounds or injury sites may require multiple grafts, which may be cost prohibitive, or preclude the use of a placental tissue graft entirely.

Accordingly, there is a need in the marketplace for an efficient manner of applying a graft over an extended and often irregularly shaped wound. One potential solution involves expanding the placental tissue via a meshing process that allows for the expansion of the tissue and an accompanying increase in the coverage area. The creation of a graft that can be spread to fit an irregular wound shape would enhance efficiency and potentially reduce the cost of treatment.

It is also to be understood that the terminology used herein is for the purpose of describing particular embodiments only, and is not intended to be limiting, since the scope of this invention will be limited only by the appended claims.

The detailed description of the invention is divided into various sections only for the reader's convenience and disclosure found in any section may be combined with that in another section. Unless defined otherwise, all technical and scientific terms used herein have the same meaning as commonly understood by one of ordinary skill in the art to which this invention belongs. All patents and publications mentioned herein disclose and describe the methods and/or materials in connection with which the publications are cited.

It is understood that where a parameter range is provided, all integers and ranges within that range, and tenths and hundredths thereof, are also provided by the embodiments. For example, "5-10%" includes 5%, 6%, 7%, 8%, 9%, and 10%; 5.0%, 5.1%, 5.2%....9.8%, 9.9%, and 10.0%; and 5.00%, 5.01%, 5.02%....9.98%, 9.99%, and 10.00%, as well as, for example, 6-9%, 5.1%-9.9%, and 5.01%-9.99%. This also applies to ratios. For example, a recited ratio range of "1:100 to 200:1" includes ratios such as 1:50, 1:1, and 100:1, along with ranges such as 1:100 to 1:1, 1:50 to 50:1, and 1:1 to 200:1.

As used herein, "about" in the context of a numerical value or range means within ±1%, ±5%, or 10% of the numerical value or range recited or claimed.

The invention illustratively disclosed herein suitably may be practiced in the absence of any element which is not specifically disclosed herein.

It must be noted that as used herein and in the appended claims, the singular forms "a", "an", and "the" include plural referents unless the context clearly dictates otherwise.

### Definitions

As used herein the following terms have the following meanings.

"Placental tissue" or "placenta" means the placental disk, and the amniotic sac, including amnion, chorion, and intermediate layer.

"Comprising" or "comprises" is intended to mean that the compositions, for example media, and methods include the recited elements, but not excluding others. "Consisting essentially of" when used to define methods, shall mean excluding other elements of any essential significance to the combination for the stated purpose. "Consisting of" shall mean excluding additional substantial method steps. Embodiments defined by each of these transition terms are within the scope of this invention.

"Dehydrated" means that the tissue has had substantially all of its water removed, (i.e. greater than 90%, greater than 95%, greater than 99%, or 100% of its water removed).

"Optional" or "optionally" means that the subsequently described event or circumstance can or cannot occur, and that the description includes instances where the event or circumstance occurs and instances where it does not.

The term "subject" as used herein is any vertebrate organism including but not limited to mammalian subjects such as humans, farm animals, domesticated pets and the like. The term "patient" may be used interchangeably with "subject."

The term "meshed" refers to placental tissue grafts which have had a plurality of cuts made in an engineered pattern, through the entirety of the thickness of the graft. These cuts form holes when the graft is expanded along at least one axis. The cuts may be of varying size, and may vary in distance from one another, or in their orientation relative to each other.

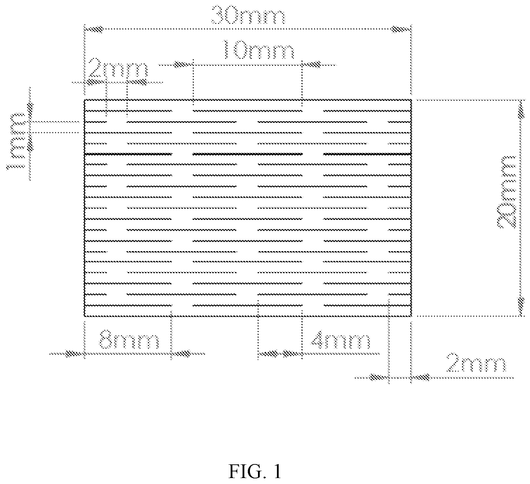

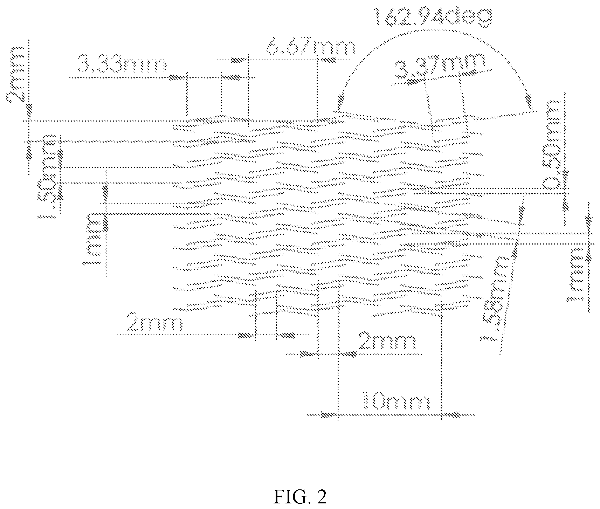

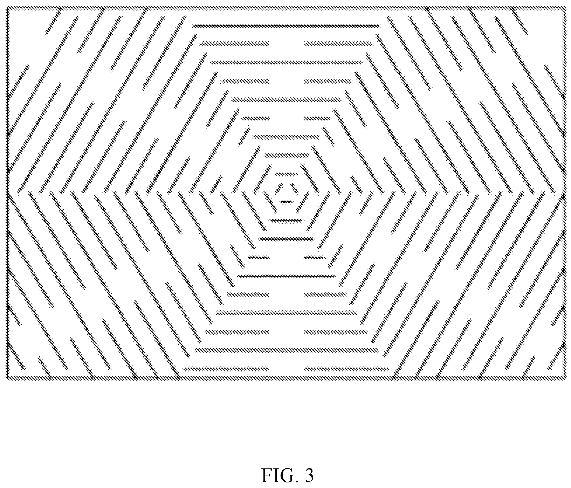

The term "engineered pattern" refers to a specific arrangement of cuts made in an placental cord graft that are not random. For example, an engineered pattern may consist of a plurality of identically-sized cuts, all parallel to each other (as in Fig. 1). Alternatively, the engineered pattern may comprise cuts which are at an angle relative to some other cuts (as in Fig. 2), or cuts which are at three or more distinct angles relative to each other (as in, e.g., Figs. 3-6).

The term "treat," with respect to a wound, means to reduce the amount of time the wound would have taken to heal in the absence of any type of medical intervention.

The term "cuts" refers to any of a number of incisions in the mesh, wherein said cuts are line cuts (or "slits"). Described but not claimed are hole punches which can be circular, ovular, rectangular, rhomboid, or irregularly-shaped holes, or combinations thereof. A "slit" is another name for a linear cut that does not remove any tissue from the graft, as would a hole punch.

The term "expandable" means the ability of the tissue graft to expand by at least 100% of its original size, thereby excluding expansion solely due to rehydration. An "expanded" tissue graft is one that has been expanded by at least 100%, and is maintained in an expanded form after the external force is removed.

Ideally, the graft can be expanded by at least 200% of its original size. In an embodiment, the graft is expanded by stretching the graft. Stretching, in this context, does not infer elastic properties. The stretching and expansion through the application of an external force over one axis is at all times to be differentiated from the slight increase in volume that the graft experiences when it is rehydrated or reconstituted prior to, or during application of, the graft to the wound site.

The term "tensile strength" means the amount of force that a graft can withstand while being stretched or expanded before failing or breaking.

The term "expansion ratio" refers to the surface area that an unexpanded meshed graft (i.e., in its natural shape) can cover compared to the same meshed graft in its expanded form. For example, a meshed graft having an expansion ratio of 1:3 will cover 3 times as much surface area in its expanded state. This is equivalent to an increase of 200% in surface area coverage.

### Detailed Description

An embodiment is a meshed, expandable, and sterile placental tissue allograft according to claim1.

The allograft comprises pre-determined cuts.

The cuts are in an engineered pattern.

In an embodiment, all of said cuts are parallel to one another.

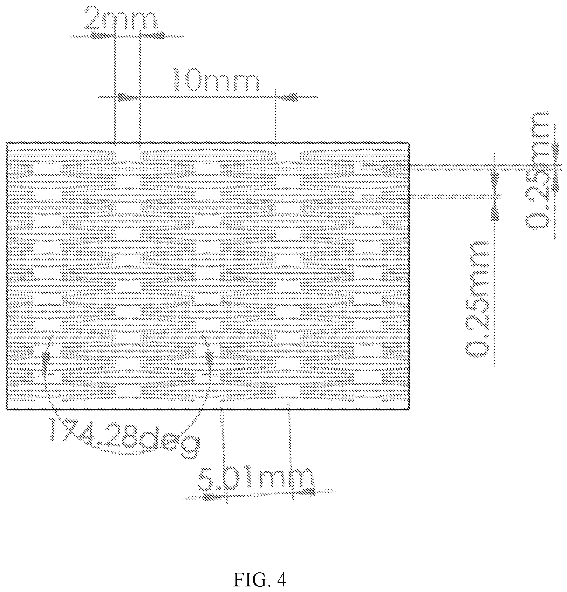

In an embodiment, said cuts are 0.1-20 mm in length. It is understood that not all cuts in a graft need to be the same size and/or shape, due to either the pattern of cuts chosen (as in, for example, Fig. 4) or due to cuts at the edge of an allograft (as in, for example, Fig. 1).

In an embodiment, the distance between two adjacent cuts is between 0.1-10 mm. It is understood that "adjacent" refers to, in various embodiments, to the distance between ends of two cuts (an end-to-end distance of 2 mm in Fig. 1) or to the distance between two cuts which run parallel to each other but are not on the same line (a side-to-side distance of 1 mm in Fig. 1).

In an embodiment, the ratio of cut length to the distance between two adjacent cuts is from 1:100 to 200:1.

In an embodiment, a first portion of said cuts are parallel to one another, a second portion of said cuts are parallel to one another, and the first portion and the second portion are oriented at an angle of 1°-180° relative to each other. An example of such a pattern is shown in Fig. 2.

In an embodiment, the allograft further comprises a third portion of cuts which is not parallel to the first portion or to the second portion. An example of such a pattern is shown in Fig. 3.

In an embodiment, the allograft comprises no more than five portions of cuts, wherein each of said no more than five portions of cuts are oriented at an angle of 1°-180° relative to each of said other portions.

In an embodiment, said cuts comprise no more than 20%, 15%, 10%, 9%, 8%, 7%, 6%, 5%, 4%, 3%, 2%, 1%, 0.5%, or 0.1% of the surface area of the allograft.

In the present invention, said cuts are slits.

In the present invention, the placental tissue allograft is dehydrated. In an embodiment, the placental tissue allograft is lyophilized.

In an embodiment, the placental tissue graft comprises amnion. In an embodiment, the placental tissue graft comprises chorion. In an embodiment, the placental tissue graft comprises amnion and intermediate layer. In an embodiment, the placental tissue graft comprises amnion layered directly on chorion. In an embodiment, the placental tissue graft comprises amnion, chorion, and intermediate layer.

In an embodiment, the sealed pouch is deoxygenated.

Also disclosed is a method of treating a subject in need thereof using the placental tissue allograft as described herein, said method comprising
i) expanding said placental tissue allograft so as to increase its surface area by at least 100%; and
ii) applying the expanded placental tissue allograft to the subject.

### Methods of Manufacture

### Initial Tissue Collection

The recovery of placenta tissue originates in a hospital, where it is collected during a Cesarean section birth. The donor, referring to the mother who is about to give birth, voluntarily submits to a comprehensive screening process designed to provide the safest tissue possible for transplantation. The screening process preferably tests for antibodies to the human immunodeficiency virus type 1 and type 2 (anti-HIV-1 and anti-HIV-2), hepatitis B surface antigens (HBsAg), antibodies to the hepatitis C virus (anti-HCV), antibodies to the human T-lymphotropic virus type I and type II (anti-HTLV-I and anti-HTLV-II), CMV, and syphilis, using conventional serological tests. The above list of tests is exemplary only, as more, fewer, or different tests may be desired or necessary over time or based upon the intended use of the grafts, as will be appreciated by those skilled in the art.

Based upon a review of the donor's information and screening test results, the donor will either be deemed acceptable or not. In addition, at the time of delivery, cultures are taken to determine the presence of, for example, *Clostridium* or *Streptococcus.* If the donor's information, screening tests, and the delivery cultures are all negative (i.e., do not indicate any risks or indicate acceptable level of risk), the donor is approved and the tissue specimen is designated as initially eligible for further processing and evaluation.

Human placentas that meet the above selection criteria are preferably individually bagged in a saline solution in a sterile shipment bag and stored in a container of wet ice for shipment to a processing location or laboratory for further processing.

### Material Check-In and Evaluation

Upon arrival at the processing center or laboratory, the shipment is opened and verified that the sterile shipment bag/container is still sealed and intact, that ice or other coolant is present and that the contents are cool, that the appropriate donor paperwork is present, and that the donor number on the paperwork matches the number on the sterile shipment bag containing the tissue. The sterile shipment bag containing the tissue is then stored in a refrigerator until ready for further processing. All appropriate forms are completed and chain of custody and handling logs are also completed.

### Gross Tissue Processing Step

When the tissue is ready to be processed further, the sterile supplies necessary for processing the placenta tissue further are assembled in a staging area in a controlled environment and are prepared for introduction into a critical environment. If the critical environment is a manufacturing hood, the sterile supplies are opened and placed into the hood using conventional sterile technique. If the critical environment is a clean room, the sterile supplies are opened and placed on a cart covered by a sterile drape. All the work surfaces are covered by a piece of sterile drape using conventional sterile techniques, and the sterile supplies and the processing equipment are placed on to the sterile drape, again using conventional sterile technique.

If the placenta tissue is collected prior to the completion or obtaining of results from the screening tests and delivery cultures, such tissue is labeled and kept in quarantine. The tissue is approved for further processing only after the required screening assessments and delivery cultures, which declare the tissue safe for handling and use, are satisfied.

Processing equipment is decontaminated according to conventional and industry-approved decontamination procedures and then introduced into the critical environment. The equipment is strategically placed within the critical environment to minimize the chance for the equipment to come in proximity to or be inadvertently contaminated by the tissue specimen.

Next, the placenta is removed from the sterile shipment bag and transferred aseptically to a sterile processing basin within the critical environment. The sterile basin contains, preferably, 18% NaCl (hyperisotonic saline) solution that is at room or near room temperature. The placenta is gently massaged to help separate blood clots and to allow the placenta tissue to reach room temperature, which will make the separation of the amnion and chorion layers from each other, as discussed hereinafter, easier. After having warmed up to the ambient temperature (after about 10-30 minutes), the placenta is then removed from the sterile processing basin and laid flat on a processing tray with the amniotic membrane layer facing down for inspection.

The placenta tissue is examined and the results of the examination are documented on a "Raw Tissue Assessment Form". The placenta tissue is examined for discoloration, debris or other contamination, odor, and signs of damage. The size of the tissue is also noted. A determination is made, at this point, as to whether the tissue is acceptable for further processing.

Next, in certain examples, if the placenta tissue is deemed acceptable for further processing, the amnion and chorion layers of the placenta tissue are then carefully separated, if desired. Alternatively, the separation of the amnion and chorion layers can be omitted and the amnion and chorion layers may be kept intact for the remainder of the rinsing and decontamination steps. The materials and equipment used in this procedure include the processing tray, 18% saline solution, sterile 4×4 sponges, and two sterile Nalgene jars. The placenta tissue is then closely examined to find an area (typically a corner) in which the amniotic membrane layer can be separated from the chorion layer. The amniotic membrane appears as a thin, opaque layer on the chorion.

With the placenta tissue in the processing tray with the amniotic membrane layer facing down, the chorion layer is gently lifted off the amniotic membrane layer in a slow, continuous motion, using care to prevent tearing of the amniotic membrane. If a tear starts, it is generally advisable to restart the separation process from a different location to minimize tearing of either layer of tissue. If the chorion layer is not needed, it may be gently scrubbed away from the amniotic membrane layer with one of the sterile 4×4 sponges by gently scrubbing the chorion in one direction. A new, sterile 4×4 sponge can be used whenever the prior sponge becomes too moist or laden with the chorion tissue. If the chorion is to be retained, then the separation process continues by hand, without the use of the sponges, being careful not to tear either the amnion layer or the chorion layer.

Care is then taken to remove blood clots and other extraneous tissue from each layer of tissue until the amniotic membrane tissue and the chorion are clean and ready for further processing. More specifically, in certain examples, the amnion and chorion tissues are placed on the processing tray and blood clots are carefully removed using a blunt instrument, a finger, or a sterile non-particulating gauze, by gently rubbing the blood until it is free from the stromal tissue of the amnion and from the trophoblast tissue of the chorion. The stromal layer of the amnion is the side of the amniotic membrane that faces the mother. In contrast, the basement membrane layer is the side of the amnion that faces the baby.

Using a blunt instrument, a cell scraper or sterile gauze, any residual debris or contamination is also removed. This step must be done with adequate care, again, so as not to tear the amnion or chorion tissues. The cleaning of the amnion is complete once the amnion tissue is smooth and opaque-white in appearance. If the amnion tissue is cleaned too much, the opaque layer can be removed. Any areas of the amnion cleaned too aggressively and appear clear will be unacceptable and will ultimately be discarded. The preceding step may be omitted if the amnion and chorion layers have not been separated.

In some examples, the intermediate tissue layer, also referred to as the spongy layer, is substantially removed from the amnion in order to expose the fibroblast layer. The term "substantially removed" with respect to the amount of intermediate tissue layer removed is defined herein as removing greater than 90%, greater than 95%, or greater than 99% of the intermediate tissue layer from the amnion. This can be performed by peeling the intermediate tissue layer from the amnion. Alternatively, the intermediate tissue layer can be removed from the amnion by wiping the intermediate tissue layer with gauze or other suitable wipe. The resulting amnion can be subsequently decontaminated using the process described below.

In some examples, the epithelium layer present on the amnion is substantially removed in order to expose the basement layer of the amnion. The term "substantially removed" with respect to the amount of epithelium removed is defined herein as removing greater than 90%, greater than 95%, or greater than 99% of the epithelial cells from the amnion. The presence or absence of epithelial cells remaining on the amnion layer can be evaluated using techniques known in the art. For example, after removal of the epithelial cell layer, a representative tissue sample from the processing lot is placed onto a standard microscope examination slide. The tissue sample is then stained using Eosin Y Stain and evaluated as described below. The sample is then covered and allowed to stand. Once an adequate amount of time has passed to allow for staining, visual observation is done under magnification.

The epithelium layer can be removed by techniques known in the art, in some embodiments. For example, the epithelium layer can be scraped off of the amnion using a cell scraper. Other techniques include, but are not limited to, freezing the membrane, physical removal using a cell scraper, or exposing the epithelial cells to nonionic detergents, anionic detergents, and nucleases. The de-epithelialized tissue is then evaluated to determine that the basement membrane has not been compromised and remains intact. This step is performed after completion of the processing step and before the tissue has been dehydrated. For example, a representative sample graft is removed for microscopic analysis. The tissue sample is place onto a standard slide, stained with Eosin Y and viewed under the microscope. If epithelium is present, it will appear as cobblestone-shaped cells.

### Chemical Decontamination Step

The amniotic membrane tissue is then placed into a sterile Nalgene jar for the next step of chemical decontamination. If the chorion is to be recovered and processed further, it too is placed in its own sterile Nalgene jar for the next step of chemical decontamination. If the chorion is not to be kept or used further, it can be discarded in an appropriate biohazard container.

Next, each Nalgene jar is aseptically filled with 18% saline solution and sealed (or closed with a top. The jar is then placed on a rocker platform and agitated for between 30 and 90 minutes, which further cleans the tissue of contaminants.

If the rocket platform was not in the critical environment (e.g., the manufacturing hood), the Nalgene jar is returned to the critical/sterile environment and opened. Using sterile forceps, the tissue is gently removed from the Nalgene jar containing the 18% hyperisotonic saline solution and placed into an empty Nalgene jar. This empty Nalgene jar with the tissue is then aseptically filled with a pre-mixed antibiotic solution. Preferably, the premixed antibiotic solution is comprised of a cocktail of antibiotics, such as Streptomycin Sulfate and Gentamicin Sulfate. Other antibiotics, such as Polymixin B Sulfate and Bacitracin, or similar antibiotics now available or available in the future, are also suitable. Additionally, it is preferred that the antibiotic solution be at room temperature when added so that it does not change the temperature of or otherwise damage the tissue. This jar or container containing the tissue and antibiotics is then sealed or closed and placed on a rocker platform and agitated for, preferably, between 60 and 90 minutes. Such rocking or agitation of the tissue within the antibiotic solution further cleans the tissue of contaminants and bacteria.

Again, if the rocker platform was not in the critical environment (e.g., the manufacturing hood), the jar or container containing the tissue and antibiotics is then returned to the critical/sterile environment and opened. Using sterile forceps, the tissue is gently removed from the jar or container and placed in a sterile basin containing sterile water or normal saline (0.9% saline solution). The tissue is allowed to soak in place in the sterile water/normal saline solution for at least 10 to 15 minutes. The tissue may be slightly agitated to facilitate removal of the antibiotic solution and any other contaminants from the tissue. After at least 10 to 15 minutes, the tissue is ready to be dehydrated and processed further.

### Lyophilization Step

The placental tissue can be laid down on the drying substrate in various configurations including single layer, bi-layer configurations that incorporate amnion and chorion, amnion and amnion, chorion and chorion, or multi-layer configurations of greater than two layers, which may be composed solely of amnion, chorion, or any combination of amnion and chorion.

Preferably, the placental tissue is placed in an individual, sealed Tyvek pouch (or other commercially available pouch) and placed into a commercially available freeze drying chamber. Any lyophilization process known to one skilled in the art may be used, so long as the placental tissue is substantially dehydrated when the lyophilization process has been completed.

Other methods may be used to adequately dehydrate the placental tissue. Such techniques may include, but are not limited to chemical dehydration, or placing the placental tissue in a low humidity/high temperature environment for an adequate period of time until optimal dehydration of the placental tissue has been achieved. Such dehydration techniques are generally well-known to those having skill in the art.

### Applying Mesh Pattern to Dehydrated Placental Tissue Graft

The dehydrated placental tissue graft is subjected to a cutting process whereby the desired mesh pattern is applied to the graft. Several methods are available to administer the desired mesh pattern to the graft. The following cutting methods are examples, and are not exclusive. There are many methods of applying cut patterns to tissue grafts that are known to those having skill in the art. One method of applying the desired pattern to the graft involves the use of a die cutting system, such as the commercially available Biocut Systems^{®} cutting die. The use of this system incorporates a custom designed die that is applied to the graft under pressure, so the that the pattern on the custom die is cut directly into the graft.

Another method of applying the desired pattern to the graft involves the use of a skin mesher, such as the commercial unit manufactured by 4Med, Ltd.^{®} "Rosenberg" Adjustable Skin Mesher. The graft can be fed through the mesher, which can be adjusted to apply different cut patterns in several different mesh ratios. Once the desired mesh ratio is set, the graft is force fed through the mesher and the cutting pattern is applied as the graft exits the meshing teeth.

The mesh pattern may also be applied through the use of a commercially available laser cutting device such as the Optek Systems^{®} laser cutting system. The schematics of the desired cut pattern can be programmed into the device, which will then use a high power laser light to cut the pattern into the graft as specified in the programmed schematics. The placental tissue graft is held in place during the cutting process on a fixture that has been custom designed for this process. Laser cutting provides several advantages over other methods, including greater flexibility with respect to desired cutting patterns, particularly with closely-spaced cuts, and no dulling of cutting edges of mechanical cutting tools. Dulling of the mechanical cutting edges may result in incomplete cuts through the entire thickness of the graft, which may have a negative impact on graft quality and expansion capabilities, resulting in an inferior product.

In an embodiment, the cuts will be in a pattern which permits stretching or expansion of the graft at least 100% by applying a force that is less than its tensile strength.

In an embodiment, the cuts will be in a pattern which permits stretching or expansion of the graft at least 100% by application of a force that is less than the graft's tensile strength. By applying a force that is less than the graft's tensile strength, the graft will maintain the desired stretched configuration without compromising its structural integrity.

In an embodiment, the cuts will be in a pattern which permits stretching or expansion of the graft at least 100% without microtears forming in the graft. It is understood that "microtears" do not include the cuts intentionally made in the graft by a die, laser cutter, or other method.

In an embodiment, the cuts will be in a pattern which permits stretching or expansion of the graft at least 100% without tears visible to the naked eye forming from cuts in the graft. It is also understood that these tears do not include the cuts intentionally made in the graft by a die, laser cutter, or other method. In an embodiment, tears form from less than 10%, 9%, 8%, 7%, 6%, 5%, 4%, 3%, 2%, or 1% of the cuts.

In an embodiment, the cuts will be in a pattern which permits stretching or expansion of the graft at least 100% without strands of the graft breaking. In an embodiment, less than 10%, 9%, 8%, 7%, 6%, 5%, 4%, 3%, 2%, or 1% of the strands break.

In some embodiments, the cuts may comprise parallel, staggered cuts, as exemplified in Fig. 1. In some embodiments, the cuts comprise two distinct portions of cuts, wherein each portion comprises closely-spaced pairs of parallel cuts, and wherein the first portion's cuts and the second portion's cuts are at an angle with respect to each other, as exemplified in Fig. 2. In some embodiments, the cuts are arranged so that the expanded graft will have a "spiderweb" pattern, as exemplified in Fig. 3. In some embodiments, the cuts are arranged in repeating patterns of straight and slanted slits, as exemplified in Figs. 4-6. For example, Figs. 4-6 show grafts prepared with staggered, repeating units, each repeating unit having a central, longer slit, on either side of which are two angled slits which meet perpendicular to the center of the central slit, so as to form a obtusely-angled V-shape.

### Packaging

After the desired mesh pattern has been applied to the dehydrated placental tissue graft, the graft is then placed within a pouch. The graft may be placed into the pouch in the presence of ambient, atmospheric air, or it can be filled with an inert gas such as nitrogen, meant to displace the ambient, atmospheric air. This pouch is then sealed and placed within another pouch, which is also sealed once the inner pouch has been introduced. The inner pouch is traditionally referred to as the sterile pouch, while the outer pouch is considered non-sterile.

### Sterilization

The inner and outer pouch along with the resulting dehydrated placental tissue grafts are subjected to a terminal sterilization step. Terminal sterilization is accomplished by exposing the dehydrated placental tissue grafts to high energy, penetrating, ionizing radiation such as electron beam or gamma irradiation while the product is in its final packaging unit.

### Reconstitution of Meshed, Dehydrated Placental Tissue Graft

In order to administer the meshed placental tissue graft to a subject, the end user must first reconstitute the graft by rehydrating it or reconstituting it with a suitable excipient. The rehydrating agent is 0.9% saline solution.

### Administration of Meshed, Dehydrated Placental Tissue Graft to a Subject

Once the placental tissue graft has been reconstituted with the desired rehydrating agent, it is then applied to the wound site. Also, the graft may be hydrated in the wound site with the rehydrating agent or blood present from wound bed preparation. The reconstituted placental tissue graft is stretched in order to achieve maximum coverage of the wound bed. The graft is stretched along a single axis by applying a force that is less than the graft's tensile strength, because application of force in excess of the tensile strength would tend to disrupt the structural integrity of the graft and cause it to rupture. One of ordinary skill in the art will understand that structural integrity is required in order for the graft to be easily applied to a site, and, in some embodiments, may be evaluated by tensile strength, tears, or strand breaks, as discussed hereinabove.

### Experimental

Grafts are prepared having a number of different cut patterns and stretched, in order to determine the suitability of the patterns for stretchable grafts.

### Experiment 1: Slit Design (Fig. 1)

The slit prototype is very similar in pattern to the slits achieved by a traditional skin graft mesher, but the slit lengths and spacing are optimized to achieve peak expansion.

### Experiment 2: Zig Zag Design (Fig. 2)

The zig zag incision pattern, when expanded, creates small holes with strips of tissue covering each small hole. This design allows for better wound coverage because the tissue strands will be diagonally positioned and very close together.

### Experiment 3: Spiderweb Design (Fig. 3)

The spiderweb design is created by positioning the slit design at different angles from the center of the graft to create a hexagonal effect.

### Experiment 4: "Evil Eye" Design (Fig. 4)

The evil eye design is a dense series of straight and slanted slits. This design allows for excellent wound coverage and expansion, but the strands of tissue will be very thin (less than 1mm thick).

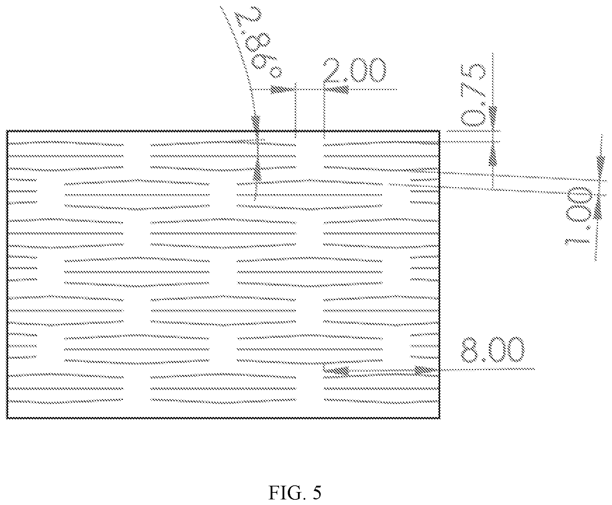

### Experiment 4.1: Evil Eye Design 2 (Fig. 5)

The evil eye design was redesigned to permit thicker strands of tissue. For the 4.1 design, the number of slits was reduced significantly.

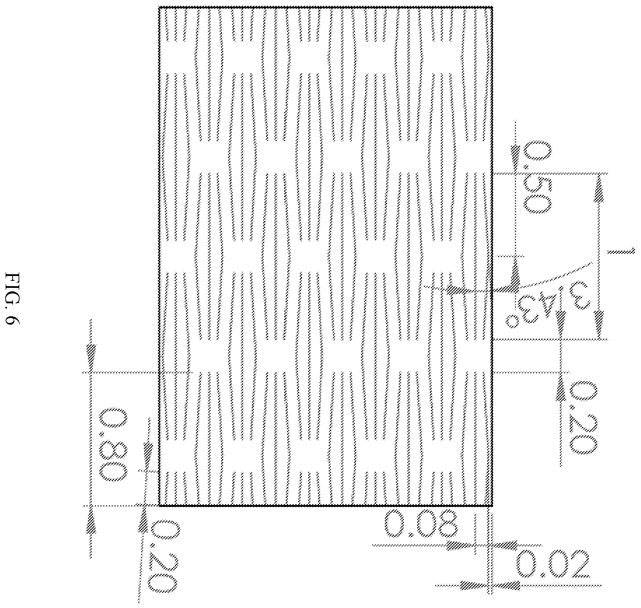

### Experiment 4.2: Evil Eye Design 3 (Fig. 6)

The evil eye design was redesigned to create thicker strands of tissue, but also allow for optimal expansion. For the 4.2 design, the number of slits was reduced slightly.

### Experiment 5: Expansion of grafts

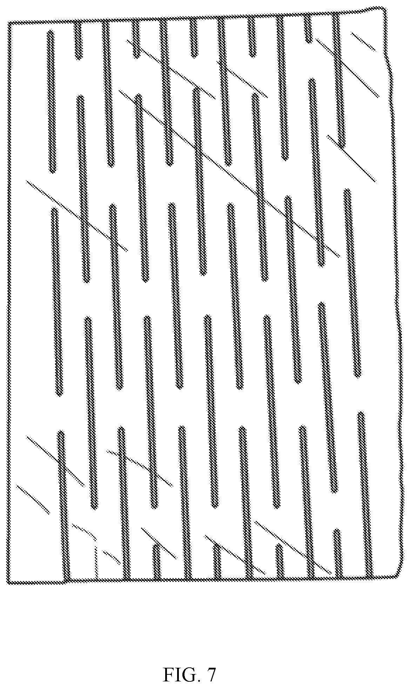

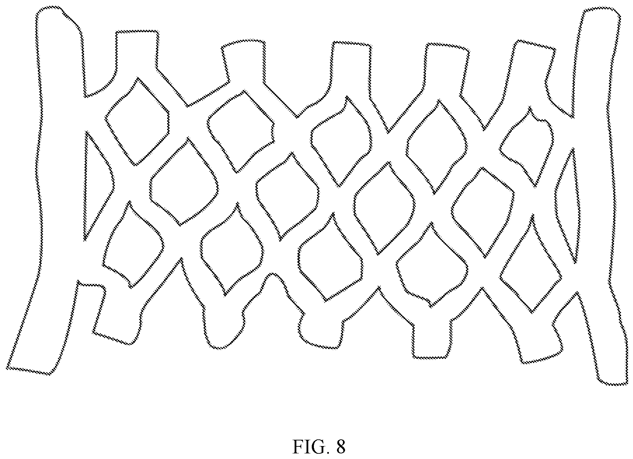

Schematic illustrations of a placental tissue graft to be prepared according to Experiment 1 are shown, both before and after expansion (Fig. 7 and Fig. 8, respectively).

Citation or identification of any reference in this application is not an admission that such reference is available as prior art.

Efforts have been made to ensure accuracy with respect to numbers used (e.g. amounts, temperature, etc.) but some experimental errors and deviations should be accounted for.

Unless defined otherwise, technical and scientific terms used herein have the same meaning as commonly understood by one of ordinary skill in the art to which this subject matter belongs, and are consistent with: Singleton et al (1994) Dictionary of Microbiology and Molecular Biology, 2nd Ed., J. Wiley & Sons, New York, NY; and Janeway, C., Travers, P., Walport, M., Shlomchik (2001) Immunobiology, 5th Ed., Garland Publishing, New York.

## Claims

1. A placental tissue allograft, wherein said placental tissue allograft is dehydrated, meshed, expandable by at least 100% of its original size when rehydrated in 0.9% saline solution, and sterile; wherein said placental tissue allograft comprises a plurality of pre-determined cuts in an engineered pattern; wherein said cuts are slits; and wherein said placental tissue allograft is contained within a sealed pouch.

2. The placental tissue allograft of claim 1, wherein all of said cuts are parallel to one another.

3. The placental tissue allograft of claim 1, wherein a first portion of said cuts are parallel to one another, a second portion of said cuts are parallel to one another, and the first portion and the second portion are oriented at an angle of 1°-180° relative to each other.

4. The placental tissue allograft of claim 1, comprising no more than five portions of cuts, wherein each of said no more than five portions of cuts are oriented at an angle of 1°-180° relative to each of said other portions.

5. The placental tissue allograft of any one of claims 1-4, wherein said cuts comprise no more than 20%, 15%, 10%, 9%, 8%, 7%, 6%, 5%, 4%, 3%, 2%, 1%, 0.5%, or 0.1% of the surface area of the allograft.

6. The placental tissue allograft of any one of claims 1-5, comprising amnion.

7. The placental tissue allograft of any one of claims 1-6, comprising chorion.

8. The placental tissue allograft of any one of claims 1-7, comprising amnion and intermediate layer.

9. The placental tissue allograft of any one of claims 1-7, comprising amnion layered directly on chorion, or amnion, chorion, and intermediate layer.

10. The placental tissue allograft of claim 1, wherein said sealed pouch is deoxygenated.

## Patentansprüche

1. Ein Plazentagewebe-Allotransplantat, wobei das Plazentagewebe-Allotransplantat dehydriert, netzartig eingeschnitten, bei Rehydrierung in 0,9%iger Kochsalzlösung um mindestens 100 % seiner ursprünglichen Größe expandierbar und steril ist; wobei das Plazentagewebe-Allotransplantat eine Vielzahl von vorgegebenen Schnitten in einem konstruierten Muster beinhaltet; wobei die Schnitte Schlitze sind; und wobei das Plazentagewebe-Allotransplantat in einem versiegelten Beutel enthalten ist.

2. Plazentagewebe-Allotransplantat gemäß Anspruch 1, wobei alle Schnitte parallel zueinander sind.

3. Plazentagewebe-Allotransplantat gemäß Anspruch 1, wobei ein erster Teil der Schnitte parallel zueinander ist, ein zweiter Teil der Schnitte parallel zueinander ist und der erste Teil und der zweite Teil in einem Winkel von 1°-180° relativ zueinander ausgerichtet sind.

4. Plazentagewebe-Allotransplantat gemäß Anspruch 1, das nicht mehr als fünf Teile von Schnitten beinhaltet, wobei jeder der nicht mehr als fünf Teile von Schnitten in einem Winkel von 1°-180° relativ zu jedem der anderen Teile ausgerichtet ist.

5. Plazentagewebe-Allotransplantat gemäß einem der Ansprüche 1-4, wobei die Schnitte nicht mehr als 20 %, 15 %, 10 %, 9 %, 8 %, 7 %, 6 %, 5 %, 4 %, 3 %, 2 %, 1 %, 0,5 % oder 0,1 % der Oberfläche des Allotransplantats ausmachen.

6. Plazentagewebe-Allotransplantat gemäß einem der Ansprüche 1-5, das Amnion beinhaltet.

7. Plazentagewebe-Allotransplantat gemäß einem der Ansprüche 1-6, das Chorion beinhaltet.

8. Plazentagewebe-Allotransplantat gemäß einem der Ansprüche 1-7, das Amnion und Zwischenschicht beinhaltet.

9. Plazentagewebe-Allotransplantat gemäß einem der Ansprüche 1-7, das direkt auf Chorion geschichtetes Amnion oder Amnion, Chorion und Zwischenschicht beinhaltet.

10. Plazentagewebe-Allotransplantat gemäß Anspruch 1, wobei der versiegelte Beutel desoxygeniert ist.

## Revendications

1. Une allogreffe de tissu placentaire, ladite allogreffe de tissu placentaire étant déshydratée, maillée, expansible d'au moins 100 % par rapport à sa taille d'origine lorsqu'elle est réhydratée dans une solution saline à 0,9 %, et stérile ; ladite allogreffe de tissu placentaire comprenant une pluralité de découpes prédéterminées selon un motif conçu ; lesdites découpes étant des fentes ; et ladite allogreffe de tissu placentaire étant contenue au sein d'une poche scellée.

2. L'allogreffe de tissu placentaire de la revendication 1, dans laquelle toutes lesdites découpes sont parallèles les unes aux autres.

3. L'allogreffe de tissu placentaire de la revendication 1, dans laquelle une première portion desdites découpes sont parallèles les unes aux autres, une deuxième portion desdites découpes sont parallèles les unes aux autres, et la première portion et la deuxième portion sont orientées selon un angle de 1° à 180° l'une par rapport à l'autre.

4. L'allogreffe de tissu placentaire de la revendication 1, comprenant au plus cinq portions de découpes, dans laquelle chacune desdites cinq portions au plus de découpes est orientée selon un angle de 1° à 180° par rapport à chacune desdites autres portions.

5. L'allogreffe de tissu placentaire de l'une quelconque des revendications 1 à 4, dans laquelle lesdites découpes ne comprennent pas plus de 20 %, 15 %, 10 %, 9 %, 8 %, 7 %, 6 %, 5 %, 4 %, 3 %, 2 %, 1 %, 0,5 %, ou 0,1 % de la superficie de l'allogreffe.

6. L'allogreffe de tissu placentaire de l'une quelconque des revendications 1 à 5, comprenant de l'amnios.

7. L'allogreffe de tissu placentaire de l'une quelconque des revendications 1 à 6, comprenant du chorion.

8. L'allogreffe de tissu placentaire de l'une quelconque des revendications 1 à 7, comprenant de l'amnios et une couche intermédiaire.

9. L'allogreffe de tissu placentaire de l'une quelconque des revendications 1 à 7, comprenant de l'amnios déposé en couche directement sur du chorion, ou de l'amnios, du chorion, et une couche intermédiaire.

10. L'allogreffe de tissu placentaire de la revendication 1, dans laquelle ladite poche scellée est désoxygénée.
